# EUROPEAN PATENT SPECIFICATION

(11) **EP 3 344 985 B1**
(45) Date of publication and mention of the grant of the patent: **01.01.2020**
(21) Application number: 16801277.1
(22) Date of filing: 20.10.2016
(51) Int. Cl.: G01N 33/00, G01N 1/22

(54) **GAS DETECTION INSTRUMENT INLET NOZZLE ASSEMBLY**
EINLASSDÜSENANORDNUNG EINES GASDETEKTIONSINSTRUMENTS
ENSEMBLE BUSE D'ENTRÉE D'INSTRUMENT DE DÉTECTION DE GAZ

(30) Priority: 29.10.2015 GB 201519143
(43) Date of publication of application: 11.07.2018
(73) Proprietor: Gas Measurement Instruments Limited, Renfrew, Renfrewshire PA4 9RG, Scotland (GB)
(72) Inventor: CORR, Andy, Renfrew Scotland PA4 9RG (GB); MCGREGOR, George, Renfrew Scotland PA4 9RG (GB)
(74) Representative: Hedges, Martin Nicholas
(86) International application number: PCT/GB2016/053280
(87) International publication number: WO 2017/072490

(56) References cited:
- EP-A1- 1 072 877
- US-A- 5 603 476
- US-A1- 2004 107 782
- US-A1- 2015 217 077

## Description

The present invention relates to gas detection instruments and more particular to inlet nozzle assemblies which form part of such gas detection instruments.

Typical gas detection instruments include a particulate filter and a hydrophobic filter in the gas inlet to help to protect the instrument from damage from moisture and foreign objects by preventing particles such as dust, and water from being drawn into the instrument by the internal pump and damaging the sensors. Due to the natural of the practical field use of these type of instruments, and because the filters are the first line of defence for the instruments, the filters are considered to be serviceable parts which will need to be replaced numerous times during the operating life of the instrument.

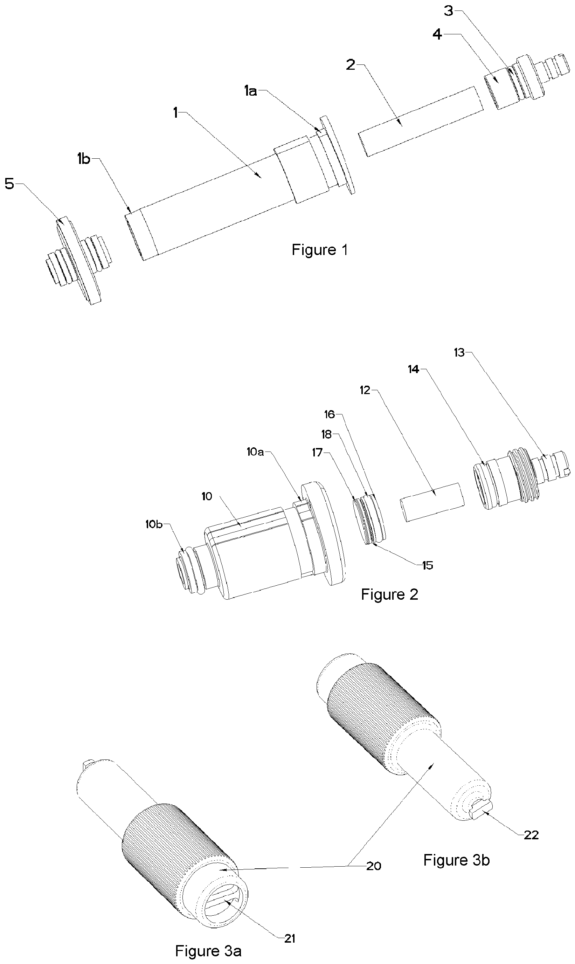

In designs known in the art, as exemplified by Figure 1, the particulate filter is integrated into the inlet assembly so that it can easily be removed and replaced externally of the instrument without having to disassemble the whole instrument, and the particulate filter can therefore easily be removed and replaced by the user in the field when required.

However, conventional design places the hydrophobic filter internally of the instrument, downstream of the inlet assembly. As a result, in order to remove and replace this filter, the whole instrument must be dis-assembled, a job which cannot reasonably be done in the field and normally necessitates returning the instrument to the manufacturer or a service centre. This is not only costly to the user but also means the user is without the instrument during the time taken to complete the service.

US2004/107782, which is considered to represent the closest prior art, discloses a gas inlet nozzle assembly comprising an inlet tube, a hydrophobic filter, a particulate filter and a retainer which retains the hydrophobic and particulate filters in the inlet tube.

Other prior art is disclosed in US 2015/217077.

According to the present invention there is provided a gas inlet nozzle according to claim 1.

A gas inlet nozzle assembly in accordance with the present invention has the advantage that by having both the hydrophobic filter and the particulate filter removably mounted in the inlet tube through the inlet end, bot filters can be removed and replaced without necessitating removal of the inlet tube and hence without requiring disassembly of the instrument. As a result, both filters can be serviced without requiring return to the manufacturer, and instead field replacement of the filters can easily be carried out by the user.

Preferably, the retainer is formed separately of the particulate filter and engages in the inlet tube upstream of the hydrophobic and particular filters. However, retainer may instead be integrated with the particular filter. The retainer furthermore preferably includes locking means which engage with complimentary locking means formed on the inlet tube, such as male and female threads, to locking secure the retainer to the inlet tube.

The retainer furthermore preferably includes seal means such as an O ring which engages with the inlet tube for effecting a fluid tight seal between the retainer and the inlet tube.

The inlet tube preferably includes a seat on its inner surface proximate the outlet end against which the hydrophobic filter engages. The hydrophobic filter further preferably includes seal means which engages with the inlet tube to effect a fluid tight seal between the hydrophobic filter and the inlet tube and thereby constrains fluid passing through the inlet assemble to pass through the hydrophobic filter. In particular, the seal means may engage with the seat of the inlet tube, but preferably the hydrophobic filter is mounted in a filter housing so as to form a hydrophobic filter assembly, a seal such as an O ring seal being provided on the outer surface of the filter housing which sealingly engages with the inner surface of the inlet tube to effect a fluid tight seal therebetween and thereby prevent fluid from passing between the housing and the inlet tube.

The present invention further provides a gas detection instrument according to claim 12.

In order that the invention may be well understood, there will now be described an embodiment thereof, given by way of example, reference being made to the accompanying drawings, in which:
Figure 1 is an exploded view of an inlet nozzle assembly according to the prior art;
Figure 2 is an exploded view of an inlet nozzle assembly according to the invention; and
Figures 3a and 3b are perspective views of a removal tool suitable for use with the inlet nozzle assembly of the invention.

Referring first to Figure 1, there is show a conventional inlet nozzle assembly known in the art. The assembly comprises an inlet tube 1 having an inlet end 1a, an outlet end 1b, and a tubular opening connecting the inlet end 1a to the outlet end 1b. A cylindrical dust filter 2 is removably engageable in the tubular opening of the inlet tube 1 from the inlet end 1a thereof, and is retained in place by an inlet connector 3 which is lockingly engageable in the inlet end 1a. The inlet connector 3 includes an o-ring seal 4 for effecting a sealing engagement with the tubular opening of the inlet tube 1.

A hydrophobic filter 5 is sealingly connected to the outlet end 1b of the inlet tube such that fluid passing through the inlet tube 1 is constrained to pass through the hydrophobic filter 5. The hydrophobic filter 5 has a connector on both sides for connecting to the inlet tube and for connecting to conduit for connection with a pump of a gas instrument (not shown). Due to the size and location of the hydrophobic filter 5, the inlet tube 1 must be removed in order to gain access to the hydrophobic filter for servicing and/or removal.

Referring now to Figure 2, there is shown an inlet nozzle assembly according to the invention. The assembly comprises an inlet tube 10 having an inlet end 10a, an outlet end 10b and a tubular opening connecting the inlet 10a to the outlet 10b. The outlet end 10b is formed as a connector for connecting the inlet tube 10 to a pump of a gas detection instrument.

The inlet nozzle 10 has a shoulder proximate its outlet end 10b which forms a seat in the tubular opening against which is engageable a hydrophobic filter assembly 15, which is insertable into the tubular opening through the inlet end 10a of the inlet tube. The hydrophobic filter assembly 15 is formed by a filter housing 16 on which is mounted a hydrophobic filter PTFE membrane 17. In the preferred embodiment, the membrane 17 has a pore size of 5 micro-meters. An o-ring seal 18 is mounted around the outside of the housing 16 which sealingly engages the surface of the tubular opening of the inlet tube 10 so as to prevent fluid passing around the outside of the filter housing 16 and thereby ensuring that all fluid passes through the hydrophobic filter 17,

A particulate filter 12 is also engageable in the tubular opening of the inlet tube behind the hydrophobic filter assembly 15 such that all fluid passes through the particulate filter 12 before reaching the hydrophobic filter 17. The particulate filter 12 is sized to engage in the end of an inlet connector 13 which is lockingly engageable in the inlet end 10a of the inlet tube so as to retain the hydrophobic filter assembly 15 and the particulate filter 12 in the inlet tube 10. In the illustrated embodiment, the inlet connector 13 has a male thread formed on its outer surface which screwingly engages with complementary female threads formed on the inner surface of the inlet tube 10 proximate the inlet end 10a therefore, but other coupling means are also possible within the scope of the invention. An O-ring seal 14 is carried on the outer surface of the inlet connector 13 for effecting a sealing engagement with the inner walls of the inlet tube 10 so as to ensure that all fluid entering the inlet tube does so through the inlet connector and is hence directed through the particulate filter 12 and subsequently the hydrophobic filter 17.

In order, then, to remove both the particulate filter 12 and the hydrophobic filter assembly 15, the user simply removes the inlet connector 13 by unscrewing it from the inlet tube 10. To facilitate such removal, the inlet connector 13 is provided with a slotted end and a removal tool 20 is provided having a removal blade 21 on one end which is engageable with the slot in the inlet connector.

Upon removal of the inlet connector 13, the particulate filter 12, which is engaged in the inside end of the inlet connector 13, is also removed from the inlet tube 10 and can then easily be pulled out of the inlet connector 13 and serviced or replaced. The hydrophobic filter assembly 15 can also then be removed from the inlet tube 10 through the inlet end 10a thereof. Due to the close fit of the seal 18 with the internal surface of the tubular opening of the inlet tube 10, the hydrophobic filter assembly 15 will be a tight fit in the inlet tube 10 and not easily removed therefrom. In order, then, to facilitate such removal, the filter housing 16 is provided with a slotted opening in its end which faces the inlet end 10a of the inlet tube 10, and the removal tool 20 has a spigot 22 formed on its second end which is of complementary size to the slotted opening of the filter housing 16 such that the spigot 22 is engageable through the slotted opening and can then be rotated in a bayonet fashion to lock the removal tool 20 to the hydrophobic filter assembly 15. Once so locked, the removal tool 20 can then be used to withdraw the hydrophobic filter assembly 15 from the inlet tube 10 for servicing or replacement of the hydrophobic filter assembly 15. The removal tool 20 may similarly be used to facilitate replacement of a hydrophobic filter assembly 15 in place proximate the outlet end 10b of the inlet tube 10 without causing damage to the hydrophobic filter 17.

It will, of course, be understood that other designs of tool are also possible within the scope of the invention.

## Claims

1. A gas inlet nozzle assembly comprising an inlet tube (10) having an inlet end (10a) and an outlet end (10b), a hydrophobic filter (15) removably engageable in the inlet tube (10) through the inlet end (10a), a particulate filter (12) removably engageable in the inlet tube (10) through the inlet end (10a), and a retainer (13) lockingly engageable in the inlet end (10a) of the inlet tube (10) so as to retain the hydrophobic filter (15) and the particulate filter (120 in the inlet tube (10), **characterised in that** the hydrophobic filter (15) includes latching means in the form of a slotted opening provided in the hydrophobic filter (15) which is engagable in a bayonet manner by a complementary spigot (22) provided on a removal tool (20) for facilitating insertion and removal of the hydrophobic filter (15) from the inlet tube (10).

2. A gas inlet nozzle assembly according to claim 1, wherein the retainer (13) is formed separately of the particulate filter (12) and engages in the inlet tube (10) upstream of the hydrophobic (15) and particulate (12) filters.

3. A gas inlet nozzle assembly according to claim 1, wherein the retainer is integrated with the particular filter (12).

4. A gas inlet nozzle assembly according to any of the preceding claims, wherein the retainer (13) includes locking means which engage with complimentary locking means (13) formed on the inlet tube (10) to locking secure the retainer (13) to the inlet tube (10).

5. A gas inlet nozzle assembly according to any of the preceding claims, wherein the retainer (13) includes seal means (14) which engages with the inlet tube (10) for effecting a fluid tight seal between the retainer (13) and the inlet tube (10).

6. A gas inlet nozzle assembly according to claim 5, wherein the seal is an O ring (14).

7. A gas inlet nozzle assembly according to any of the preceding claims, wherein the inlet tube (10) includes a seat on its inner surface proximate the outlet end (10b) against which the hydrophobic filter (15) engages.

8. A gas inlet nozzle assembly according to claim 7, wherein the hydrophobic filter (15) includes seal means (18) which engages with the inlet tube (10) to effect a fluid tight seal between the hydrophobic filter (15) and the inlet tube (10) and thereby constrains fluid passing through the inlet tube (10) to pass through the hydrophobic filter (15).

9. A gas inlet nozzle assembly according to claim 8, wherein the seal means (18) of the hydrophobic filter (15) engages with the seat of the inlet tube (10).

10. A gas inlet nozzle assembly according to claim 8, wherein the hydrophobic filter (15) includes a filter housing (16), a seal (18) being provided on the outer surface of the filter housing (16) which sealingly engages with the inner surface of the inlet tube (10) to effect a fluid tight seal therebetween and thereby prevent fluid from passing between the housing (16) and the inlet tube (10), the slotted opening associated with the hydrophobic filter (15) being provided in filter housing (16).

11. A gas inlet nozzle assembly according to claim 9 or claim 10, wherein the seal is an O ring seal (18).

12. A gas detection instrument comprising a housing containing a sensor, an inlet, a pump for delivering gas from the inlet to the sensor, a gas inlet nozzle assembly according any of the preceding claims provided in the fluid flow path between the inlet and the pump, and a removal tool (20) having latching means in the form of a spigot (22) provided thereon which are complementary to the slotted opening in the hydrophobic filter (15) such that the removal tool (20) is engageable with the hydrophobic filter (15) in a bayonet manner to facilitate insertion and removal of the hydrophobic filter (15) from the inlet tube (10).

13. A gas detection instrument according to claim 12, wherein the removal tool (20) includes a removal blade (21) which is engageable with a slot in the inlet connector (13) to facilitate removal of the inlet connector (13).

## Patentansprüche

1. Gaseinlassdüsenanordnung, umfassend ein Einlassrohr (10) mit einem Einlassende (10a) und einem Auslassende (10b), einen hydrophoben Filter (15), der durch das Einlassende (10a) hindurch lösbar mit dem Einlassrohr (10) in Eingriff gebracht werden kann, einen Partikelfilter (12), der durch das Einlassende (10a) hindurch lösbar mit dem Einlassrohr (10) in Eingriff gebracht werden kann, und einem Halter (13), der mit dem Einlassende (10a) des Einlassrohrs (10) in Verriegelungseingriff gebracht werden kann, sodass der hydrophobe Filter (15) und der Partikelfilter (120) im Einlassrohr (10) gehalten werden, **dadurch gekennzeichnet, dass** der hydrophobe Filter (15) Rastmittel in Form einer in dem hydrophoben Filter (15) bereitgestellten geschlitzten Öffnung beinhaltet, die durch einen komplementären Zapfen (22), der an einem Entnahmewerkzeug (20) bereitgestellt ist, bajonettartig in Eingriff genommen werden kann, um ein Einsetzen und Lösen des hydrophoben Filters (15) von dem Einlassrohr (10) zu erleichtern.

2. Gaseinlassdüsenanordnung nach Anspruch 1, wobei der Halter (13) getrennt vom Partikelfilter (12) ausgebildet ist und in das Einlassrohr (10) stromaufwärts des hydrophoben (15) und des Partikelfilters (12) in Eingriff kommt.

3. Gaseinlassdüsenanordnung nach Anspruch 1, wobei der Halter im Partikelfilter (12) integriert ist.

4. Gaseinlassdüsenanordnung nach einem der vorhergehenden Ansprüche, wobei der Halter (13) Verriegelungsmittel beinhaltet, die komplementäre Verriegelungsmittel (13), die an dem Einlassrohr (10) ausgebildet sind, in Eingriff nehmen, um den Halter (13) am Einlassrohr (10) verriegelnd zu sichern.

5. Gaseinlassdüsenanordnung nach einem der vorhergehenden Ansprüche, wobei der Halter (13) Dichtungsmittel (14) beinhaltet, die das Einlassrohr (10) in Eingriff nehmen, um eine fluiddichte Abdichtung zwischen dem Halter (13) und dem Einlassrohr (10) zu erzeugen.

6. Gaseinlassdüsenanordnung nach Anspruch 5, wobei die Dichtung ein O-Ring (14) ist.

7. Gaseinlassdüsenanordnung nach einem der vorhergehenden Ansprüche, wobei das Einlassrohr (10) eine Befestigungsfläche an seiner Innenfläche nahe dem Auslassende (10b) beinhaltet, mit der der hydrophobe Filter (15) in Eingriff ist.

8. Gaseinlassdüsenanordnung nach Anspruch 7, wobei der hydrophobe Filter (15) Dichtungsmittel (18) beinhaltet, die mit dem Einlassrohr (10) in Eingriff stehen, um eine fluiddichte Abdichtung zwischen dem hydrophoben Filter (15) und dem Einlassrohr (10) zu erzeugen, und ein durch das Einlassrohr (10) strömende Fluid somit zwingen, durch den hydrophoben Filter (15) zu strömen.

9. Gaseinlassdüsenanordnung nach Anspruch 8, wobei die Dichtungsmittel (18) des hydrophoben Filters (15) mit der Befestigungsfläche des Einlassrohrs (10) in Eingriff stehen.

10. Gaseinlassdüsenanordnung nach Anspruch 8, wobei der hydrophobe Filter (15) ein Filtergehäuse (16) beinhaltet, wobei an der Außenfläche des Filtergehäuses (16) eine Dichtung (18) bereitgestellt ist, die mit der Innenfläche des Einlassrohrs (10) abdichtend in Eingriff steht, um eine fluiddichte Abdichtung dazwischen zu erzeugen und ein Strömen von Fluid zwischen dem Gehäuse (16) und dem Einlassrohr (10) somit zu verhindern, wobei die geschlitzte Öffnung, die mit dem hydrophoben Filter (15) verbunden ist, im Filtergehäuse (16) bereitgestellt ist.

11. Gaseinlassdüsenanordnung nach Anspruch 9 oder Anspruch 10, wobei die Dichtung eine O-Ring-Dichtung (18) ist.

12. Gaserkennungsinstrument, umfassend ein Gehäuse, das einen Sensor, einen Einlass, eine Pumpe zum Befördern von Gas vom Einlass zum Sensor, eine Gaseinlassdüsenanordnung nach einem der vorhergehenden Ansprüche, die im Fluidströmungsweg zwischen dem Einlass und der Pumpe bereitgestellt ist, und ein Entnahmewerkzeug (20) mit darauf bereitgestellten Rastmitteln in Form eines Zapfens (22) enthält, die komplementär zu der geschlitzten Öffnung in dem hydrophoben Filter (15) sind, sodass das Entnahmewerkzeug (20) mit dem hydrophoben Filter (15) bajonettartig in Eingriff gebracht werden kann, um ein Einsetzen und Entnehmen des hydrophoben Filters (15) aus dem Einlassrohr (10) zu erleichtern.

13. Gaserkennungsinstrument nach Anspruch 12, wobei das Entnahmewerkzeug (20) eine entnehmbare Klinge (21) beinhaltet, die mit einem Schlitz in dem Einlassanschluss (13) in Eingriff gebracht werden kann, um ein Entfernen des Einlassanschlusses (13) zu durchzuführen.

## Revendications

1. Ensemble de buse d'admission de gaz comprenant un tube d'admission (10) ayant une extrémité d'admission (10a) et une extrémité de refoulement (10b), un filtre hydrophobe (15) pouvant être engagé de façon amovible dans le tube d'admission (10) à travers l'extrémité d'admission (10a), un filtre à particules (12) pouvant être engagé de façon amovible dans le tube d'admission (10) à travers l'extrémité d'admission (10a) et un élément de retenue (13) pouvant être engagé de manière verrouillée dans l'extrémité d'admission (10a) du tube d'admission (10) de façon à retenir le filtre hydrophobe (15) et le filtre à particules (120) dans le tube d'admission (10) ; **caractérisé en ce que** le filtre hydrophobe (15) comprend un moyen d'enclenchement sous la forme d'une ouverture à fente prévue dans le filtre hydrophobe (15) et dans laquelle peut s'engager, à la manière d'une baïonnette, un ergot complémentaire (22) prévu sur un outil de retrait (20) afin de faciliter l'insertion et le retrait du filtre hydrophobe (15) vis-à-vis du tube d'admission (10).

2. Ensemble de buse d'admission de gaz selon la revendication 1, dans lequel l'élément de retenue (13) est formé séparément du filtre à particules (12) et s'engage dans le tube d'admission (10) en amont des filtres hydrophobe (15) et à particules (12).

3. Ensemble de buse d'admission de gaz selon la revendication 1, dans lequel l'élément de retenue est intégré au filtre à particules (12) .

4. Ensemble de buse d'admission de gaz selon l'une quelconque des revendications précédentes, dans lequel l'élément de retenue (13) comprend des moyens de verrouillage qui s'engagent avec des moyens de verrouillage complémentaires (13) formés sur le tube d'admission (10) pour arrimer par verrouillage l'élément de retenue (13) au tube d'admission (10).

5. Ensemble de buse d'admission de gaz selon l'une quelconque des revendications précédentes, dans lequel l'élément de retenue (13) comprend un moyen formant joint (14) qui s'engage avec le tube d'admission (10) pour former un joint étanche aux fluides entre l'élément de retenue (13) et le tube d'admission (10).

6. Ensemble de buse d'admission de gaz selon la revendication 5, dans lequel le joint est un joint torique (14).

7. Ensemble de buse d'admission de gaz selon l'une quelconque des revendications précédentes, dans lequel le tube d'admission (10) comprend un siège sur sa surface interne, à proximité de l'extrémité de refoulement (10b), contre lequel s'engage le filtre hydrophobe (15).

8. Ensemble de buse d'admission de gaz selon la revendication 7, dans lequel le filtre hydrophobe (15) comprend un moyen formant joint (18) qui s'engage avec le tube d'admission (10) pour former un joint étanche aux fluides entre le filtre hydrophobe (15) et le tube d'admission (10), pour ainsi forcer le fluide traversant le tube d'admission (10) à passer à travers le filtre hydrophobe (15).

9. Ensemble de buse d'admission de gaz selon la revendication 8, dans lequel le moyen formant joint (18) du filtre hydrophobe (15) s'engage avec le siège du tube d'admission (10).

10. Ensemble de buse d'admission de gaz selon la revendication 8, dans lequel le filtre hydrophobe (15) comprend un boîtier de filtre (16), un joint (18) étant prévu sur la surface externe du boîtier de filtre (16) qui s'engage de manière étanche avec la surface interne du tube d'admission (10) pour former un joint étanche aux fluides entre eux et ainsi empêcher le passage de fluide entre le boîtier (16) et le tube d'admission (10), l'ouverture à fente associée au filtre hydrophobe (15) étant prévue dans le boîtier de filtre (16).

11. Ensemble de buse d'admission de gaz selon la revendication 9 ou la revendication 10, dans lequel le joint est un joint torique (18) .

12. Instrument de détection de gaz comprenant un boîtier contenant un capteur, une admission, une pompe destinée à fournir au capteur du gaz provenant de l'admission, un ensemble de buse d'admission de gaz selon l'une quelconque des revendications précédentes prévu sur le trajet d'écoulement de fluide entre l'admission et la pompe, et un outil de retrait (20) présentant un moyen d'enclenchement sous la forme d'un ergot (22) prévu sur celui-ci et complémentaire de l'ouverture à fente ménagée dans le filtre hydrophobe (15), de sorte que l'outil de retrait (20) peut s'engager avec le filtre hydrophobe (15) à la manière d'une baïonnette afin de faciliter l'insertion et le retrait du filtre hydrophobe (15) vis-à-vis du tube d'admission (10).

13. Instrument de détection de gaz selon la revendication 12, dans lequel l'outil de retrait (20) comprend une lame de retrait (21) qui peut s'engager avec une fente ménagée dans le raccord d'admission (13) pour faciliter le retrait du raccord d'admission (13) .
